# EUROPÄISCHE PATENTSCHRIFT

(11) **EP 1 987 761 B1**
(45) Veröffentlichungstag und Bekanntmachung des Hinweises auf die Patenterteilung: **23.10.2019**
(21) Anmeldenummer: 07116982.5
(22) Anmeldetag: 21.09.2007
(51) Int. Cl.: A61B 5/00

(54) **Schlauchförmiger Sensor zum Nachweis eines Analyten**
Tube-like sensor for proving an analyte
Capteur tubulaire destiné à la détection d'un analyte

(30) Priorität: 03.05.2007 EP 07107436
(43) Veröffentlichungstag der Anmeldung: 05.11.2008
(73) Patentinhaber: F. Hoffmann-La Roche AG, 4070 Basel (CH); Roche Diabetes Care GmbH, 68305 Mannheim (DE)
(72) Erfinder: Schwind, Karin, 67105 Schifferstadt (DE); Haar, Hans-Peter, 69168 Wiesloch (DE); Fuerst, Otto, 68519 Viernheim (DE)
(74) Vertreter: Altmann Stößel Dick Patentanwälte PartG mbB

(56) Entgegenhaltungen:
- EP-A- 0 320 109
- EP-A- 0 645 160
- GB-A- 1 465 417
- US-A- 5 299 571
- US-A1- 2007 027 370
- US-A1- 2007 027 384

## Beschreibung

### Gebiet der Erfindung

Die Erfindung betrifft einen subkutanen Sensor zur Ermittlung einer Konzentration mindestens eines Analyten in einem Medium, insbesondere einem Körpergewebe und/oder einer Körperflüssigkeit. Weiterhin betrifft die Erfindung ein Sensorsystem sowie ein Verfahren zur Herstellung eines implantierbaren Sensors. Derartige Sensoren oder Sensorsysteme werden insbesondere im Bereich der Medizintechnik eingesetzt, beispielsweise um elektrochemisch eine Konzentration von Blutglukose, Triglyceriden, Laktat oder anderen Analyten zu bestimmten.

### Stand der Technik

Die Bestimmung der Blutglukosekonzentrationen sowie eine entsprechende Medikation ist für Diabetiker ein essentieller Bestandteil des Tagesablaufs. Dabei muss die Blutglukosekonzentration schnell und einfach mehrmals am Tag (typischerweise zwei bis sieben Mal) bestimmt werden, um gegebenenfalls entsprechende medizinische Maßnahmen ergreifen zu können. In vielen Fällen erfolgt dabei eine Medikation mittels automatischer Systeme, insbesondere so genannte Insulinpumpen.

Um den Tagesablauf des Diabetikers nicht mehr als nötig einzuschränken, werden häufig entsprechend mobile Geräte eingesetzt, welche einfach zu transportieren und zu handhaben sein sollten, so dass eine Messung der Blutglukosekonzentration, beispielsweise am Arbeitsplatz oder auch in der Freizeit, problemlos erfolgen kann. Derzeit sind verschiedene mobile Geräte am Markt, welche teilweise nach unterschiedlichen Messmethoden und unter Verwendung unterschiedlicher Diagnoseverfahren funktionieren. Ein erstes Messverfahren beruht beispielsweise auf einer elektrochemischen Messmethode, wobei eine Blutprobe, welche vom Patienten beispielsweise durch Perforieren einer Hautschicht mittels einer Lanzette dem Körpergewebe entnommen wird, auf eine mit Enzymen und Mediatoren beschichtete Elektrode appliziert wird. Entsprechende Teststreifen für derartige elektrochemische Messverfahren sind beispielsweise in US 5,286,362 beschrieben. Andere bekannte Messmethoden verwenden optische Messverfahren, welche beispielsweise darauf beruhen, dass die zu detektierende Substanz (Analyt) mit bestimmten Nachweisreagenzien reagieren kann, wobei eine Farbänderung des Reaktionsgemisches eintritt. Systeme zum Nachweis derartiger Farbreaktionen und somit zum Nachweis der entsprechenden Analyten sind beispielsweise aus CA 2,050,677 bekannt.

Die beschriebenen Nachweisverfahren beruhen also überwiegend darauf, dass ein Patient zunächst eine entsprechende Probe der zu untersuchenden Körperflüssigkeit entnimmt (wobei es sich beispielsweise um eine Blutprobe oder eine Urinprobe handelt) und diese dann entsprechend mittels der Testvorrichtung untersucht. Dieses Verfahren beinhaltet jedoch verschiedene Nachteile. So ist zum einem dieses Verfahren äußerst aufwändig und setzt mehrere Handhabungsschritte voraus. So muss beispielsweise eine Lanzette bereitgestellt und gespannt werden, anschließend mittels dieser Lanzette eine Hautschicht perforiert werden, dann ein so erzeugter Blutstropfen auf einen Teststreifen aufgetragen werden und anschließend dieser Teststreifen mittels eines entsprechenden Gerätes ausgewertet werden. Für viele Patienten, insbesondere ältere Menschen und Kinder, sind diese Handhabungsschritte oftmals nur schwer durchzuführen, da die Patienten beispielsweise in ihrer motorischen Fähigkeit und ihrem Sehvermögen eingeschränkt sind. Weiterhin lassen sich diese Verfahrensschritte nur in wenigen Fällen diskret durchführen, so dass beispielsweise ein Schutz der Privatsphäre des Patienten bei einer Messung am Arbeitsplatz nur unzureichend gewahrt ist. Auch kann eine Fehlbedienung des Messverfahrens leicht zu falschen Messwerten führen, mit teilweise fatalen Folgen einer auf den Messergebnissen aufbauenden falschen Medikation.

Aus dem Stand der Technik sind daher Systeme bekannt, welche in ein Körpergewebe implantiert werden können und welche beispielsweise kontinuierlich Messwerte liefern. So offenbart beispielsweise US 6,892,085 B2 ein gekapseltes Glukosesensorsystem, welches einen Glukosesensor und eine Schutzkapselung umfasst. Dabei sind drei Elektroden, eine Arbeitselektrode, eine Gegenelektrode und eine Referenzelektrode vorgesehen, welche auf einer Seite eines dünnen Substrats aufgebracht sind. Zur besseren Implantierbarkeit ist diese Elektrodenanordnung in eine Hohlnadel eingebracht, welche in Körpergewebe eingestochen wird.

Auch US 5,591,139 offenbart ein implantierbares Mikronadelsystem, mittels dessen für Diagnosezwecke Substanzen aus lebendem Gewebe entnommen werden können. Dabei wird ein geätztes dreidimensionales Substrat verwendet.

In US 2007/0027384 A1 werden Systeme und Verfahren für eine kontinuierliche Analyt-Überwachung beschrieben. Dabei werden verschiedene Ausführungsformen koaxialer Aufbauten implantierbarer Sensoren beschrieben, in denen verschiedene Elektroden elektrochemischer Sensoren koaxial übereinandergelagert sind und in unterschiedlichen axialen Bereichen enden, so dass jeweils eine aktive Fläche einer Elektrode freiliegt. Aufgrund des unterschiedlichen Durchmessers dieser koaxialen Elektroden sind diese einzelnen Elektrodenflächen jedoch in ihren Eigenschaften unterschiedlich und in ihrer Anzahl begrenzt. Zudem wird zwar in US 2007/0027384 A1 das koaxiale Design im Gegensatz zu planaren Sensorkonstruktionen als besonders unempfindlich gegenüber Biegungen angesehen, wobei jedoch der massive Aufbau an sich eine hohe Steifigkeit der Sensoren bedingt und wobei Biegungen sogar zu einer Veränderung der aktiven Elektrodenflächen und somit zu einer massiven Beeinflussung der Messwerte führen können. Alternativ wird in US 2007/0027384 A1 ein stabförmiger Aufbau vorgeschlagen, bei welchem spiralförmige Elektroden um eine stabförmige Basiselektrode gewickelt sind. Dieser Aufbau ist mit dem Nachteil verbunden, dass die exakte Lage der einzelnen Elektroden undefiniert sein kann, da sich die spiralförmigen Elektroden entlang einer Längsachse der Sensoren verschieben können. Zudem ist die Anzahl der derart ausgestalteten Elektroden begrenzt, so dass beispielsweise eine Mittelung über von mehreren Elektroden erzielte Messwerte mit diesem Aufbau nur schwer realisierbar ist.

Auch US 5,299,571 offenbart eine Vorrichtung zur Implantierung von in-vivo-Sensoren. Dabei wird eine flexible Röhre mit einem doppelten Innenlumen eingesetzt. Eines dieser Innenlumen nimmt eine Nadel auf, welcher der Implantation dient und anschließend entfernt werden kann. Das zweite Innenlumen nimmt einen Sensor auf, der über ein Fenster in der Röhre mit dem Gewebe in Kontakt steht WO2006/124759 offenbart eine Vorrichtung und ein Verfahren gemäß dem kennzeichnenden Teil der Ansprüche 1 und 12.

Insgesamt sind die aus dem Stand der Technik bekannten implantierbaren Sensoren bezüglich ihres Aufbaus und ihrer Herstellung äußerst aufwendig. Geht man davon aus, dass es sich bei diesen Sensoren um nur für kurze Zeit (typischerweise circa eine Woche) verwendbare Einwegsensoren handelt, so wird deutlich, dass die bei den aus dem Stand der Technik bekannten Sensoren eingesetzten Verfahren derartigen Anforderungen an Einwegartikel nicht gerecht werden.

So ist beispielsweise für die Herstellung des aus US 5,591,139 bekannten Sensors ein aufwändiges Mikrostrukturierungsverfahren erforderlich, insbesondere ein lithographisches Verfahren. Derartige Verfahren sind jedoch mit der Herstellung kostengünstiger Einwegartikel nicht vereinbar. Auch zur Herstellung des aus US 6,892,085 B2 bekannten Sensors sind aufwändige Strukturierungsverfahren erforderlich, da die Elektrodenpads sorgfältig strukturiert werden müssen. Angesichts der geringen Größe dieser Elektroden sind dafür ebenfalls lithographische Verfahren erforderlich, was die Kosten zur Herstellung derartiger Sensoren wiederum in die Höhe treibt.

Auch die aus US 5,299,571 bekannte Konstruktion ist technisch aufwändig, da hier nicht nur eine Röhre mit zwei Innenlumen hergestellt werden muss, sondern da auch noch in eines dieser Innenlumen ein gewöhnliches, jedoch von seinen Dimensionen her miniaturisiertes Sensorelement eingebracht werden muss.

Auch sind lithographische Verfahren, insbesondere das mit diesen Verfahren verbundene Ätzen von Metallschichten, nicht immer so zuverlässig, wie dies für die Herstellung medizintechnischer Produkte erforderlich ist. Insbesondere kann es vorkommen, dass einzelne Elektroden noch durch "Brücken" miteinander verbunden sind, so dass die Funktionalität der Sensoren aufgrund von Herstellungsproblemen leicht beeinträchtig oder sogar ganz verhindert sein kann.

### Aufgabe der Erfindung

Aufgabe der Erfindung ist es somit, einen Sensor zur Verfügung zu stellen, welcher einfach und kostengünstig mittels eines zuverlässigen Herstellungsverfahrens herstellbar ist und welcher die Nachteile der aus dem Stand der Technik bekannten Sensoren und Verfahren nach Möglichkeit vermeidet. Der Sensor soll zudem gut implantierbar sein, im implantierten Zustand gut verträglich sein.

### Darstellung der Erfindung

Diese Aufgabe wird durch die Erfindung mit den Merkmalen der unabhängigen Ansprüche gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt der Beschreibung gemacht.

Es wird ein implantierbarer Sensor vorgeschlagen, welcher zur Ermittlung einer Konzentration mindestens eines Analyten in einem Medium geeignet ist. Insbesondere kann es sich bei diesem Medium um ein Körpergewebe, wie beispielsweise subkutanes Gewebe und/oder um eine Körperflüssigkeit handeln. Neben den eingangs genannten medizintechnischen Anwendungen sind weitere Anwendungen denkbar, insbesondere Anwendungen im Bereich des "Continuous Monitoring" (CM) in verschiedenen Bereichen der Medizintechnik, Naturwissenschaften und der Technik. Derartige Anwendungen finden sich neben dem Bereich der Medizintechnik beispielsweise in der Qualitätskontrolle (beispielsweise in der Lebensmittelchemie), in der Umweltanalytik oder in anderen Bereich der Technik oder Naturwissenschaften, in welchen elektrochemisch eine Konzentration eines Analyten bestimmt werden muss.

Der implantierbare Sensor weist vorzugsweise eine Implantationsnadel sowie ein die Implantationsnadel umgebendes flexibles, schlauförmiges Sensorelement auf. Unter dem Begriff "schlauchförmig" ist hier und im Folgenden ein Element zu verstehen, welches im Wesentlichen die Gestalt eines Hohlzylinders mit einem Innenlumen aufweist. Das Innenlumen ist vorzugsweise zentral angeordnet, so dass ein symmetrischer Hohlzylinder bevorzugt ist. Weiterhin ist vorzugsweise ein einzelnes Innenlumen vorgesehen, im Gegensatz zu beispielsweise dem in US 5,299,571 beschriebenen Aufbau, in welchem das eigentliche planare Sensorelement nicht im Innenlumen eines Hohlzylinders angeordnet ist, sondern in einer separaten, von zentralen Innenlumen getrennt ausgebildeten "Tasche" angeordnet ist. Hierdurch wird erfindungsgemäß die Symmetrie des Sensorelements erheblich gesteigert, und das Sensorelement kann flexibler und unempfindlicher gegenüber Biegungen gestaltet werden. Durch die zentrale Anordnung des Innenlumens stellt der Hohlzylinder somit keine einfache Ummantelung eines Sensorelements dar, sondern bildet ein Trägermaterial des Sensorelements. Hierdurch kann auch auf zusätzliche Ummantelungsmaterialien verzichtet werden, und der gesamte Aufbau kann stark vereinfacht werden. Zudem wird die Implantation bzw. Insertion des Sensorelements in Gewebeschichten mit einer atraumatischen Nadelspitze erleichtert.

Die Wand des Hohlzylinders soll insbesondere biegsam und dehnbar sein, beispielsweise um auf die Implantationsnadel aufgezogen werden zu können und/oder um im Körpergewebe keine Verletzungen hervorzurufen. Die Wand des Hohlzylinders kann daher insbesondere verformbar, d.h. plastisch und/oder elastisch, sein und kann entlang einer Längsachse des Hohlzylinders, welche eine gekrümmte Bahn annehmen kann, im Wesentlichen konstant sein, kann jedoch auch mit variierender Dicke versehen sein, beispielsweise mit einer über die Längserstreckung des schlauchförmigen Elements um nicht mehr als 10% variierenden Dicke. Auch der Querschnitt des Innenlumens ist über die Längserstreckung des schlauchförmigen Elements vorzugsweise im Wesentlichen konstant, kann jedoch auch variieren, beispielsweise um nicht mehr als 30%, wobei insbesondere im Bereich stärkerer Krümmungen jedoch auch stärkere Querschnittsverengungen auftreten können.

Das schlauchförmige Sensorelement weist seinerseits einen flexiblen, schlauchförmigen Körper auf. Unter "flexibel" ist dabei eine Verformbarkeit des Körpers unter üblichen, im implantierten Zustand auftretenden Kräften zu verstehen, was Verletzungen des umgebenden Gewebes im implantierten Zustand vermeiden soll. Dadurch wird der Komfort für den Patienten erheblich erhöht. Beispielsweise kann als Material für den flexiblen schlauchförmigen Körper ein Kunststoffmaterial verwendet werden, beispielsweise ein thermoplastisches und/oder elastomeres Kunststoffmaterial wie zum Beispiel Silikon, Polyethylen, Polyester oder Polyimid.

Im Gegensatz zu dem in US 5,299,571 offenbarten Sensoraufbau ist bei dem erfindungsgemäßen implantierbaren Sensor kein zusätzliches Sensorelement in den schlauchförmigen Körper eingeschoben und durch ein Fenster mit der Gewebeflüssigkeit in Kontakt, sondern dieser schlauchförmige Körper bildet selbst die Basis für das Sensorelement. Zu diesem Zweck sind mindestens zwei Elektrodenringe zur elektrochemischen Bestimmung der Konzentration des Analyten auf den schlauchförmigen Körper aufgebracht.

Unter dem Begriff "Elektrodenring" sind dabei nicht notwendigerweise gleichmäßig kreisförmig ausgestaltete und voll umlaufende Ringe zu verstehen, auch wenn voll umlaufende, d.h. sich über einen ganzen Umfang des schlauchförmigen Sensorelements erstreckende Elektrodenringe bevorzugt sind. Unter einem "Elektrodenring" ist dabei jedoch eine Elektrodenfläche zu verstehen, welche sich zumindest teilweise über einen Umfang des schlauchförmigen Sensorelements erstreckt, im Gegensatz beispielsweise zu rein eben ausgestalteten Elektrodenflächen.

So kann der schlauchförmige Körper wie auch das schlauchförmige Sensorelement in seinem Querschnitt auch von einer Kreisform abweichen und beispielsweise eine vieleckige Form annehmen. Entsprechend nehmen dann vorzugsweise auch die Elektrodenringe, welche umfangsseitig auf den schlauchförmigen Körper aufgebracht sind, diese Querschnittsformen an.

Auch in ihrer Längserstreckung muss es sich bei diesen "Ringen" nicht notwendigerweise um Ringe mit geraden und zueinander parallelen Begrenzungen, welche entlang des Umfangs des schlauchförmigen Körpers verlaufen, handeln, sondern es können auch gezackte Elektrodenringe oder Elektrodenringe vorgesehen sein, deren Ränder in einigen Bereichen schräg zur Achse des schlauchförmigen Körpers verlaufen.

Hinsichtlich ihrer Geschlossenheit über die Umfangsfläche hinweg müssen die Elektrodenringe nicht notwendigerweise vollständig geschlossen sein, d.h. sich nicht über den vollen Umfang des schlauchförmigen Sensorelements erstrecken. Die Elektrodenringe können beispielsweise auch einen oder mehrere Spalte aufweisen. Vorzugsweise erstrecken sich die Elektrodenringe jedoch über den größten Teil des Umfangs, d.h. über vorzugsweise mindestens 60% des Umfangs, besonders bevorzugt 80% des Umfangs und im Idealfall über mindestens 95% des Umfangs des schlauchförmigen Sensorelements oder sogar über den gesamten Umfang (d.h. ohne Spalt).

Zur Kontaktierung der Elektrodenringe sind vorzugsweise in dem schlauchförmigen Körper elektrisch und/oder galvanisch voneinander isolierte Zuleitungen für die mindestens zwei Elektrodenringe eingebettet. Vorzugsweise handelt es sich bei diesen Zuleitungen um Drähte. Diese Drähte können bei der Herstellung des schlauchförmigen Körpers beispielsweise durch Koextrusion mit dem Material des Körpers erzeugt werden und/oder in den Körper eingebracht werden.

Diese Zuleitungen sind vorzugsweise durch den Körper zumindest teilweise durchdringende elektrische Durchkontaktierungen elektrisch mit den Zuleitungen verbunden. Beim Herstellen dieser Durchkontaktierungen werden vorzugsweise zunächst ein oder mehrere Durchkontaktierungswege (englisch: vias) in mindestens einer Materialschicht zwischen den Elektrodenringen und den Zuleitungen erzeugt. Anschließend wird dieser Durchkontaktierungsweg zumindest teilweise mit mindestens einem elektrisch leitfähigen Material gefüllt. Zum Herstellen der Durchkontaktierungen bzw. Durchkontaktierungswege kann beispielsweise mindestens eines der folgenden Verfahren eingesetzt werden: ein mechanisches Bohrverfahren, insbesondere ein herkömmliches Bohrverfahren; ein Laserbohrverfahren; ein Laserablationsverfahren; ein Ätzverfahren. Auch andere Verfahren, die bei der Leiterplattenherstellung eingesetzt werden, sind denkbar. Als leitfähiges Material zur Füllung der Durchkontaktierungswege kann beispielsweise mindestens eines der folgenden Materialien eingesetzt werden: ein Metall; einen Leitkleber; eine Leitpaste, insbesondere eine Leitpaste mit mindestens einem Bindermaterial und mindestens einem Anteil leitfähiger Partikel, wobei vorzugsweise als leitfähige Partikel Kohlenstoffpartikel, insbesondere Mikro- oder Nanopartikel, Metallpartikel oder Mischungen dieser Partikel verwendet werden. Auch ähnliche leitfähige Materialien oder Materialkombinationen lassen sich einsetzen. Besonders bewährt haben sich Leitpasten, wobei vorzugsweise Leitpasten mit mindestens einem Bindermaterial und mindestens einem Anteil leitfähiger Partikel eingesetzt werden. Als Bindermaterial können beispielsweise Acryl-Kleber verwendet werden, welche vorzugsweise mit leitfähigen Partikeln gefüllt sind. Als besonders geeignet haben sich Kohlenstoffpartikel, insbesondere Mikro- oder Nanopartikel erwiesen. Auch Metallpartikel oder Mischungen der genannten Partikelarten können eingesetzt werden. Weiterhin können die Pasten zusätzlich Lösungsmittel enthalten, wobei als Lösungsmittel beispielsweise Ethylenglykol- oder Propylenglykol-Derivate (d.h. Ester oder Ether hiervon) in Frage kommen, wie sie bei vielen in der Elektrotechnik benötigten Pasten eingesetzt werden. Bevorzugt sind dabei aufgrund der harmlosen Abbauwege Derivate von Propan-1,2-diol.

Die Art und der Aufbau der Elektrodenringe können an den nachzuweisenden Analyten angepasst werden. Insbesondere können die Elektrodenringe einfache Metallelektroden umfassen. Alternativ oder zusätzlich können die Elektrodenringe auch derart aufgebaut sein, dass diese jeweils mindestens einen dem schlauchförmigen Körper zugewandten elektrisch leitfähigen Kontaktring (beispielsweise hergestellt aus einer Metallschicht und/oder einer Leitpaste) und jeweils mindestens eine auf dem Kontaktring aufgebrachte Funktionsschicht umfassen.

Dabei können die mindestens zwei Elektroden insbesondere mindestens eine Arbeitselektrode und mindestens eine weitere Elektrode umfassen. Die mindestens eine weitere Elektrode kann beispielsweise als Gegenelektrode und/oder als Referenzelektrode betrieben werden. Insbesondere sollte die mindestens eine Gegenelektrode ein zum Redoxverhalten der mindestens einen Arbeitselektrode umgekehrtes Redoxverhalten aufweisen. Eine Gegenelektrode und eine Referenzelektrode können auch als gemeinsame Elektrode ausgebildet sein, vorzugsweise als eine gemeinsame Elektrode, deren Elektrodenfläche größer ist als die Fläche der mindestens einen Arbeitselektrode. Insgesamt können die Flächen der Elektrodenringe angepasst werden an die gewünschten Stromdichten.

Aus dem Stand der Technik sind verschiedene Beispiele für die Verwendung von Elektrodenmaterialien (d.h. sowohl für die Kontaktringe als auch für die Funktionsschichten) für elektrochemische Messverfahren bekannt. So können beispielsweise Elektroden mit Enzymen oder anderen chemischen Hilfsstoffen beschichtet werden, welche spezifisch für den nachzuweisenden Analyten sind. Beispielsweise kann zum Nachweis von Glukose Glukoseoxidase (GOD) eingesetzt werden, welche Glukose in Glukonolacton umwandelt. Die dabei frei werdenden Ladungsträger werden nachgewiesen. Um diesen Nachweis zu ermöglichen, werden üblicherweise überspannungsreduzierende Materialien eingesetzt, welche als Ladungsvermittler zwischen der Funktionsschicht und den Kontaktringen dienen. Viele dieser überspannungsreduzierenden Materialien sind jedoch gesundheitsschädlich. Insbesondere haben sich dabei Mediatoren neben den Enzymen als toxisch erwiesen, so dass eine Immobilisierung dieser überspannungsreduzierenden Materialien für einen Einsatz in implantierbaren Sensoren in vielen Fällen erforderlich ist. Zur Immobilisierung kann beispielsweise eine kovalente Bindung an die Kontaktringe und/oder an eine Schicht der Elektrode erfolgen, beispielsweise eine Metallschicht. Eine zweite Möglichkeit besteht darin, dass überspannungsreduzierende Material in eine unlösliche Schicht zu integrieren, welche unlöslich ist in einer den implantierbaren Sensor im implantierten Zustand umgebenden Flüssigkeit, insbesondere in einer Körperflüssigkeit. Dies kann beispielsweise durch die Verwendung von Braunstein erfolgen, welcher als Paste als Bestandteil der Funktionsschichten eingesetzt werden kann und welcher dann nach dem Trocknen im Wesentlichen unlöslich ist.

Als Mediatoren können beispielsweise Nitrosoaniline, Hexocyanoferrat, Ferrocene oder andere bekannte Mediatoren eingesetzt werden. Neben Braunstein lassen sich auch andere Materialien einsetzen.

Neben der beschriebenen Ausgestaltung der einen beziehungsweise mehreren als Arbeitselektrode fungierenden Elektrodenringe kann auch die Ausgestaltung der als Referenzelektrode und/oder als Gegenelektrode fungierenden Elektrodenringe auf verschiedene Weisen erfolgen. So sollte die mindestens eine Referenzelektrode ein Elektrodensystem mit einem elektrochemischen Potential aufweisen, welches sich in einem Arbeitsbereich des implantierbaren Sensors nicht oder nur unwesentlich ändert, wenn sie stromlos betrieben wird. Auf diese Weise ist sichergestellt, dass die Referenzelektrode als wirkliche Referenz wirkt, mit deren Potential das elektrochemische Potential der mindestens einen Arbeitselektrode verglichen werden kann.

Grundsätzlich lassen sich auch für die Referenzelektrode eine Mehrzahl von Materialien und/oder Materialkombinationen einsetzen. Als besonders vorteilhaft hat sich dabei ein Silber/Silberchlorid (Ag/AgCl)-Elektrodensystem erwiesen. Auch andere Elektrodensysteme sind prinzipiell einsetzbar, sind jedoch weniger üblich, wie zum Beispiel Hg₂Cl₂-Elektrodensysteme.

Je nach Beschaltung können jeweils ein oder mehrere Elektrodenringe, die als Arbeitselektrode ausgelegt sind, mit einem oder mehreren Elektrodenringen, die als Referenzelektrode ausgelegt sind, zusammenwirken. Zusätzlich können, je nach Beschaltung, bei dieser Zusammenwirkung auch eine oder mehrere als Gegenelektroden geschaltete Elektrodenringe eingeschlossen sein. Dementsprechend ist es bevorzugt, wenn der implantierbare Sensor eine Anzahl von Elektrodenringen aufweist, welche ein ganzzahliges Vielfaches von Drei oder ein ganzzahliges Vielfaches von Zwei ist. In diesem Fall bilden die zusammengehörenden Gruppen der Elektrodenringe jeweils vorzugsweise voneinander unabhängige Einzelsensoren. Vorzugsweise liegt die Gesamtzahl der Elektrodenringe im Bereich zwischen (einschließlich) 2 und 21 und besonders bevorzugt im Bereich zwischen (einschließlich) 6 und 15.

Auch die mindestens eine Gegenelektrode kann auf eine Vielzahl von verschiedenen Weisen ausgestaltet sein. Dabei sollte jedoch gewährleistet sein, dass die mindestens eine Gegenelektrode ein zu einem Redoxverhalten der mindestens einen Arbeitselektrode umgekehrtes Redoxverhalten gegenüber der umgebenden Flüssigkeit aufweist. Wenn also an der Arbeitselektrode eine Oxidation stattfindet, so sollte an der Gegenelektrode eine Reduktion stattfinden, und umgekehrt. Prinzipiell lassen sich reine Metalle als Gegenelektroden einsetzen, wie beispielsweise Platin. In diesem Fall könnte auf die zusätzliche Funktionsschicht auf dem leitfähigen Kontaktring (in diesem Fall ein Platinring) verzichtet werden. Dies hat jedoch den Nachteil, dass an derartigen Metallelektroden typischerweise eine Gasbildung auftritt, beispielsweise eine Bildung von Wasserstoff oder Sauerstoff. Eine derartige Gasbildung ist jedoch bei implantiertem Sensor im Körpergewebe unerwünscht. Insofern ist es auch hier wiederum von Vorteil, wenn ein Elektrodensystem, insbesondere ein Redox-Elektrodensystem, eingesetzt wird, bei welchem eine Gasbildung vermieden wird. Insbesondere lässt sich auch hier wieder ein Ag/AgCl-Elektrodensystem einsetzen. Dabei wird beispielsweise AgCl reduziert. Daraus ist ersichtlich, dass die Gegenelektrode im Betrieb des Sensors verbraucht wird. Ist die Gegenelektrode aufgebraucht, findet wiederum häufig eine Gasbildung statt, so dass der implantierbare Sensor im Betrieb in der Regel eine begrenzte Lebensdauer aufweist. Dementsprechend kann es beispielsweise auch von Vorteil sein, wenn der als Gegenelektrode ausgestaltete Elektrodenring von seiner Fläche her größer ausgestaltet ist als der beziehungsweise die als Arbeitselektrode ausgestalteten Elektrodenringe.

Die Art und Weise der Aufbringung der Elektrodenringe beziehungsweise der Kontaktringe und/oder der Funktionsschichten kann jeweils an die verwendeten Materialien angepasst werden. Insbesondere sollte diese Aufbringung derart erfolgen, dass die Elektrodenringe beziehungsweise deren Teilschichten strukturiert aufgebracht werden, d.h. nicht großflächig, sondern bereits im Wesentlichen in ihrer gewünschten Ringform. Werden beispielsweise reine Metalle als Elektrodenmaterialien eingesetzt, so lassen sich zum Beispiel Folienverfahren (zum Beispiel Laminieren) einsetzen oder auch nasschemische Verfahren, physikalische Aufbringungsverfahren (physical vapour deposition, PVD), zum Beispiel Aufdampfen oder Aufsputtern oder auch chemische Aufbringungsverfahren (chemical vapour deposition, CVD). Braunstein oder Kohlenstoff (MnO₂/C) lässt sich beispielsweise als Beschichtung aufbringen, beispielsweise als Pastenbeschichtung oder als galvanische Beschichtung. Hierzu können unterschiedliche, dem Fachmann bekannte Pastenbeschichtungsverfahren eingesetzt werden, beispielsweise Siebdruck, Rakeln, Düsenbeschichtung, Ink-Jet-Verfahren, Piezo- oder Thermo-Jet-Verfahren, Kontaktdruckverfahren (zum Beispiel Tampondruckverfahren, Offset-Druckverfahren) oder auch elektrostatische Druckverfahren eingesetzt werden. Dabei kann beispielsweise ein Enzym (zum Beispiel GOD) bereits mit der Paste vermischt werden, so dass das Enzym und Braunstein in einem Schritt aufgebracht werden können. Alternativ kann auch zunächst Braunstein aufgebracht werden, woraufhin in einem nachfolgenden Schritt das Enzym aufgebracht wird, zum Beispiel durch Aufdispensieren oder durch einen weiteren nasschemischen Schritt. Insgesamt ist es bevorzugt, wenn beim Aufbringen der Kontaktringe und/oder der Funktionsschichten jeweils mindestens eines der folgenden Verfahren zum strukturierten Aufbringen einer Schicht verwendet wird: ein Laminierverfahren; ein Sprühverfahren; ein Inkjet-Verfahren, insbesondere ein Piezo- oder Thermojet-Verfahren; ein Kontaktdruckverfahren, insbesondere ein Tampondruckverfahren, ein Offset-Verfahren; ein elektrostatisches Druckverfahren.

Entsprechend werden auch die anderen Elektrodenringe beziehungsweise anderen Elektrodenschichten durch entsprechende Verfahren, wie beispielsweise Sprühverfahren, Ink-Jet-Verfahren oder die anderen der genannten Verfahren oder weitere Verfahren oder Verfahrenskombinationen aufgebracht. Typische Schichtdicken der Elektrodenringe liegen beispielsweise im Bereich von 10 µm, können jedoch bis in den Bereich von 100 bis einigen 100 µm liegen. Auch dünnere Elektrodenschichten sind denkbar.

Neben den beschriebenen Verfahren zum Aufbringen der Elektrodenringe, bei welchen die Elektrodenringe unmittelbar auf den schlauchförmigen Körper aufgebracht werden, lassen sich auch (wie oben durch das Laminierverfahren bereits angedeutet) indirekt Aufbringverfahren einsetzen. Beispielsweise können beim Aufbringen der Kontaktringe und/oder der Funktionsschichten einzelne oder mehrere der Schichten zumindest teilweise auf ein flexibles Trägersubstrat aufgebracht werden, wobei anschließend das flexible Trägersubstrat auf den schlauchförmigen Körper aufgebracht wird. Beispielsweise kann dieses Aufbringen durch Verkleben erfolgen, und/oder es kann auch eine Halterung durch zusätzliche Halteelemente oder durch eine Presspassung erfolgen.

Da die Elektrodenringe beziehungsweise üblicherweise für die Funktionsschichten verwendete Materialien teilweise toxisch sein können und beispielsweise als Zellgifte wirken können, ist es bevorzugt, wenn die Elektrodenringe auf der dem schlauchförmigen Körper abgewandten Seite zumindest teilweise mit mindestens einer Membranschicht umgeben sind. Dabei sollte die mindestens eine Membranschicht zumindest teildurchlässig sein für den mindestens einen nachzuweisenden Analyten. Beispielsweise kann die mindestens eine Membranschicht eine Durchlässigkeit für Glukose, Laktat, Triglyceride und/oder weitere Analyten aufweisen. Dabei sollte jedoch vorteilhafterweise die mindestens eine Membranschicht undurchlässig sein für die bei dem elektrochemischen Messverfahren eingesetzten Hilfschemikalien, beispielsweise für eingesetzte Enzyme, welche auf eine oder mehrere der Elektroden aufgebracht sind (zum Beispiel Glukoseoxidase). Somit gewährleistet die Membranschicht auch, dass diese Hilfschemikalien nicht in das Körpergewebe gelangen und dort Schaden anrichten können.

Die mindestens eine Membranschicht kann beispielsweise den Bereich, in welchem die Elektrodenringe aufgebracht sind, umschließen. Beispielsweise kann die mindestens eine Membranschicht ein Polymer umfassen oder ein Polymer sein, beispielsweise ein Polyurethan. Auch ein mehrschichtiger Membranschichtaufbau ist möglich. Beispielsweise können für das Aufbringen der Membranschicht wiederum nasschemische Verfahren, wie beispielsweise Tauchverfahren und/oder Sprühverfahren eingesetzt werden oder auch andere bekannte Beschichtungsverfahren.

Die Implantationsnadel kann Bestandteil des Sensors sein oder kann als separates Element gelagert und bezogen werden, um dann später, unmittelbar vor Implantation, in das schlauchförmige Sensorelement eingeschoben werden. Die Implantationsnadel wird vorzugsweise nach Implantation des Sensors in das Körpergewebe zumindest teilweise aus dem schlauchförmigen Körper herausgezogen und ist dementsprechend vorzugsweise gleitbar in diesem schlauchförmigen Körper gelagert. Statt eines vollständigen Herausziehens kann auch ein lediglich teilweises Herausziehen erfolgen, beispielsweise gefolgt von einem Abknicken der Implantationsnadel.

Um Irritationen beim Implantieren zu vermeiden und dennoch eine gute Implantation zu gewährleisten, hat es sich als vorteilhaft erwiesen, als Implantationsnadel eine Akupunkturnadel zu verwenden, insbesondere eine Akupunkturnadel mit einem Durchmesser zwischen 0,4 mm und 0,7 mm. Auch andere Durchmesser sind jedoch möglich, beispielsweise Durchmesser zwischen 0,2 mm und 0,35 mm. Vorzugsweise wird eine Implantationsnadel verwendet, welche eine atraumatische Spitze aufweist, d.h. eine Spitze ohne scharfe Kanten, die beispielsweise in der US 5,299,571 zu erkennen sind. Alternativ oder zusätzlich kann die Nadel auch eine Spitze mit einem biokompatiblen, enzymatisch abbaubaren Material aufweisen, welches derart mit dem Körpergewebe und/oder einer Körperflüssigkeit reagiert, dass sich auch in diesem Fall die Spitze nach Implantation in das Körpergewebe zumindest teilweise verrundet. Alternativ oder zusätzlich wäre auch eine Verrundung durch thermische Einwirkung möglich, wenn ein Material verwendet würde, welches sich bei Einwirkung der Körpertemperatur zumindest teilweise verrunden würde.

Der schlauchförmige Körper weist vorzugsweise eine Länge zwischen 3 mm und 50 mm, insbesondere zwischen 10 mm und 25 mm und besonders bevorzugt bei 18 mm auf. Vorzugsweise werden dabei schlauchförmige Körper mit einem Außendurchmesser zwischen 0,3 mm und 1,5 mm, beispielsweise zwischen 0,5 und 1,5 mm, und besonders bevorzugt bei 0,8 mm verwendet. Entsprechend dem Außendurchmesser des schlauchförmigen Körpers und dem Außendurchmesser der Akupunkturnadel werden Wandstärken zwischen 0,05 mm und 0,4 mm bevorzugt, insbesondere Wandstärken bei 0,2 mm. Derartige Wandstärken erlauben noch beispielsweise die Verwendung von Extrusionsverfahren und Koextrusionsverfahren unter gleichzeitiger Einbettung der Zuleitungen und unter Gewährleistung der elektrischen Isolierung dieser Zuleitungen.

Die Elektrodenringe sind vorzugsweise in ihrer Erstreckung längs der Sensorachse klein gegenüber der Länge des schlauchförmigen Körpers. Daher unterliegen diese vorzugsweise nicht den Einschränkungen, welchen planare Sensoren aufgrund der bei diesen Sensoren aufgebrachten Elektroden beim Biegen unterliegen, was eine hohe Flexibilität und Biegbarkeit des planaren Sensors begünstigt. Die Elektrodenringe können beispielsweise in Längsrichtung (d.h. in einer Richtung parallel zur Sensorachse) eine Breite aufweisen, welche zwischen 1% und 10% der Länge des schlauchförmigen Körpers liegt, besonders bevorzugt im Bereich von 5%. Anstelle der Gesamtlänge des schlauchförmigen Körpers könnte auch die Interaktionslänge des Sensors verwendet werden, also derjenige Teil des schlauchförmigen Sensors, welcher nach dem Implantieren innerhalb des Gewebes angeordnet ist. Alternativ oder zusätzlich kann die Breite der Elektrodenringe auch bevorzugt als Vielfaches des Durchmessers des schlauchförmigen Körpers angesehen werden und liegt vorzugsweise zwischen 20% und 200%, besonders bevorzugt bei 100%, des Durchmessers.

Weiterhin sind die Elektrodenringe vorzugsweise so angeordnet, dass diese einen Abstand aufweisen, welcher ebenfalls vorzugsweise im Bereich zwischen 1% und 10%, besonders bevorzugt bei 5%, der Länge des schlauchförmigen Körpers liegt. In Vielfachen des Durchmessers des schlauchförmigen Körpers angegeben, liegt der bevorzugte Abstand bei und der Abstand zwischen benachbarten Ringen zwischen 20% und 200%, und besonders bevorzugt bei 100% des Durchmessers des schlauchförmigen Körpers.

Dabei können grundsätzlich die Breiten der Elektrodenringe und deren Abstände individuell gewählt werden, das heißt für jeden Elektrodenring individuell, beispielsweise unterschiedlich zu den anderen Elektrodenringen. Alternativ kann jedoch auch eine äquidistante Anordnung der Elektrodenringe sowie, alternativ oder zusätzlich, auch eine gleichmäßige Breite gewählt werden. Die Elektrodenringe weisen weiterhin vorzugsweise alle einen zumindest innerhalb der Fertigungstoleranzen identischen Durchmesser (beispielsweise Innendurchmesser) auf, können jedoch prinzipiell auch unterschiedlich in ihren Durchmessern ausgestaltet werden. Der Durchmesser kann beispielsweise im Wesentlichen dem Außendurchmesser des Sensorelements entsprechen. Die Elektrodenringe weisen vorzugsweise keinerlei Überlappung untereinander in Richtung der Längsachse des Sensorelements auf, wie dies beispielsweise aus dem koaxialen Aufbau der US 2007/0027384 A1 der Fall ist. Hierdurch lässt sich ein hochsymmetrischer Sensoraufbau erzeugen, welcher auch bei geringfügigen Biegungen nahezu keinerlei Änderungen der Elektrodenflächen aufweist. Die Anzahl der Elektrodenringe kann derart gewählt werden, dass eine Vielzahl von Messpunkten entlang der Sensorachse abgefragt werden kann, so dass auch lokale Konzentrationsunterschiede auf einfache Weise ausgemittelt werden können. Beispielsweise können zehn oder mehr Elektrodenringe vorgesehen sein. Hierdurch unterscheidet sich der Aufbau beispielsweise vorteilhaft von dem in US 2007/0027384 A1 beschriebenen spiralförmigen Elektrodenaufbau, welcher eine derartige Ausgestaltung nicht erlaubt.

Die Breite der Elektrodenringe ist vorzugsweise derart ausgestaltet, dass die gesamte Breite dieser Ringe als aktive Fläche für die Elektrodenringe elektrochemisch nutzbar ist. Hierdurch unterscheiden sich die Elektrodenringe ebenfalls durch den in US 2007/0027384 A1 beschriebenen koaxialen Aufbau. Die Nutzung der gesamten Elektrodenringe als aktive Elektrodenfläche bewirkt, dass auch bei Biegungen des Sensorelements keine wesentlichen Änderungen der aktiven Elektrodenflächen auftreten können. Jeder Elektrodenring hat vorzugsweise seine eigene elektrische Ableitung bzw. Kontaktierung, so dass die Elektrodenringe galvanisch gut voneinander getrennt sind. Somit entstehen auch zwischen den einzelnen Elektrodenringen keine elektrochemischen Wechselwirkungen oder sogar Kurzschlüsse, wie es bei dem in US 2007/0027384 A1 beschriebenen koaxialen Aufbau bei einer Durchfeuchtung der trennenden Schichten möglich ist.

An einem der Spitze der Implantationsnadel abgewandeten Ende des Sensorelements können elektrische Kontakte zur Kontaktierung der Elektrodenringe vorgesehen sein bzw. bei der Herstellung des Sensorelements erzeugt werden. Diese elektrischen Kontakte können ähnlich ausgestaltet sein wie die oben beschriebenen Kontaktringe, so dass beispielsweise zu jeder Zuleitung ein separater Elektrodenkontaktring vorgesehen ist. Zur Verbindung zwischen den Zuleitungen und den elektrischen Kontakten kann beispielsweise auf die oben beschriebenen Verfahren hinsichtlich der Verbindung zwischen den Elektrodenringen und den Zuleitungen verwiesen werden. Beispielsweise können hierzu auch wiederum zunächst Durchkontaktierungswege geschaffen werden, die dann ganz oder teilweise mit einem elektrisch leitfähigen Material gefüllt werden.

Alternativ oder zusätzlich zur Verwendung einzelner Kontaktierungsringe für jede Zuleitung können die Kontakte auch auf einen gemeinsamen, beispielsweise segmentierten Anschlusskontaktring auf die Oberfläche des Sensorelements herausgeführt werden. Um die elektrischen Kontakte elektrisch mit Signalen zu beaufschlagen oder entsprechende Messsignale von diesen abzugreifen, kann auf übliche Methoden zurückgegriffen werden, beispielsweise auf Methoden, welche aus der Display-Technologie bekannt sind (zum Beispiel Leitgummis, anisotrop leitfähige Kunststoffe, Flexverbinder oder ähnliches).

Um die sich entlang der Längsachse des schlauchförmigen Sensorelements im schlauchförmigen Körper erstreckenden Zuleitungen auf der Seite der Nadelspitze abzuschließen, kann zusätzlich an dem der Spitze der Implantationsnadel zugewandten Ende des schlauchförmigen Sensorelements dieses mit einer elektrischen Isolierung abgeschlossen sein, welche die einzelnen Zuleitungen voneinander und vom Körpergewebe trennt. Diese elektrische Isolierung kann beispielsweise einen isolierenden Lack umfassen. Zur Aufbringung dieser Isolierung beziehungsweise dieses Lacks können beispielsweise wiederum Sprüh- und/oder Tauchverfahren oder auch andere Verfahren eingesetzt werden.

Der implantierbare Sensor und das beschriebene Verfahren zu dessen Herstellung zeichnen sich gegenüber den oben beschriebenen, bekannten implantierbaren Sensorsystemen durch die Verwendung einfacher und kostengünstiger Verfahrensschritte aus. Dadurch lassen sich die Sensoren kostengünstig herstellen. Gleichzeitig wirkt sich jedoch insbesondere die mögliche redundante Ausgestaltung der Elektrodenringe und der dadurch möglichen gleichzeitigen Verwendung mehrerer voneinander unabhängiger Sensoreinheiten auf ein und demselben schlauchförmigen Sensorelement äußerst positiv auf die Zuverlässigkeit der Messsignale aus. Lokale Konzentrationsunterschiede lassen sich dadurch ausmitteln, und die Langzeitstabilität der Sensorsysteme lässt sich erheblich verbessern.

Der implantierbare Sensor lässt sich insbesondere einsetzen in einem erfindungsgemäßen Sensorsystem, welches neben einem oder mehreren der oben beschriebenen implantierbaren Sensoren in einer der beschriebenen Ausgestaltungen weiterhin eine Ansteuer- und Auswerteeinheit umfasst. Diese wird elektrisch mit den Elektrodenringen verbunden und ist eingerichtet, um eine elektrochemische Messung zur Bestimmung der Konzentration des Analyten durchzuführen. Zu diesem Zweck kann die Ansteuer- und Auswerteeinheit entsprechende Spannungs- und/oder Stromquellen sowie Spannungs- und/oder Strommessvorrichtungen (jeweils eine oder mehrere) umfassen. Weiterhin können zusätzliche Elemente umfasst sei, wie beispielsweise Auswerteelemente, wie beispielsweise ein Mikrocomputer, Bedienelemente und Ausgabeelemente, wie beispielsweise ein Display. Derartige Ansteuer- und Auswerteeinheiten sind dem Fachmann bekannt mit Ausnahme dessen, dass im vorliegenden Fall auf analoge Weise, je nach Ausgestaltung des Sensorelements, die Signale von mehr als einer Gruppe von zusammengehörenden Elektrodenringen verarbeitet werden sollten.

Das Sensorsystem kann insbesondere derart ausgestaltet sein, dass, wie oben beschrieben, mindestens einer der Elektrodenringe als Arbeitselektrode geschaltet wird, und mindestens ein Elektrodenring als Gegenelektrode und/oder als Referenzelektrode. Alternativ oder zusätzlich können auch separate Elektrodenringe als Referenzelektrode und Gegenelektrode vorgesehen sein. Vorzugsweise sind die Elektrodenringe jeweils alternierend beschaltet, so dass beispielsweise auf eine Gegenelektrode eine Referenzelektrode und anschließend eine Arbeitselektrode folgt, anschließend wieder eine Gegenelektrode, eine Referenzelektrode und eine Arbeitselektrode usw.. Werden lediglich zwei Arten von Elektrodenringen verwendet, so kann beispielsweise auf einer Arbeitselektrode eine Gegenelektrode folgen, dann wieder eine Arbeitselektrode und eine Gegenelektrode usw.. Letztere Ausgestaltung ist insbesondere bei einer Beschaltung bevorzugt, bei welcher ausschließlich eine Wechselstrom-Messmethode eingesetzt wird. Auch alternative Ausgestaltungen sind jedoch denkbar, beispielsweise Ausgestaltungen, bei denen sich mehrere Sensoreinheiten eine Referenz- und/oder Gegenelektrode teilen.

Auf diese Weise lässt sich mittels eines oder mehrerer der vorgeschlagenen implantierten Sensoren ein Sensorsystem zusammenstellen, welches dem Patienten über einen längeren Zeitraum hinweg (beispielsweise eine Woche) konstant und zuverlässig Messdaten liefert, beispielsweise über eine Blutglukosekonzentration. Das Sensorsystem kann diese Informationen auch an entsprechende weitere Geräte oder Personen weitergeben, beispielsweise durch Datenübertragung zu einem Computersystem und/oder durch Datenübertragung an ein Medikationssystem (wie beispielsweise eine Insulinpumpe). Das System ist kostengünstig herstellbar und in seiner Betriebsweise äußerst zuverlässig.

### Ausführungsbeispiele

Weitere Einzelheiten und Merkmale ergeben sich aus den nachfolgenden Beschreibungen der Ausführungsbeispiele in Verbindung mit den Unteransprüchen. Hierbei können die jeweiligen Merkmale für sich alleine oder zu mehreren in Kombination miteinander verwirklicht sein.

Im Einzelnen zeigt:
- Figur 1: ein Ausführungsbeispiel eines erfindungsgemäßen Sensorsystems;
- Figuren 2A und 2B: verschiedene Darstellungen eines Ausführungsbeispiels der Kontaktierung von Elektrodenringen;
- Figur 3: ein erstes Ausführungsbeispiel einer Spitze eines implantierbaren Sensors;
- Figur 4: ein zweites Ausführungsbeispiel der Spitze eines implantierbaren Sensors; und
- Figur 5: einen schematischen Ablaufplan eines Ausführungsbeispiels eines erfindungsgemäßen Verfahrens zur Herstellung eines implantierbaren Sensors.

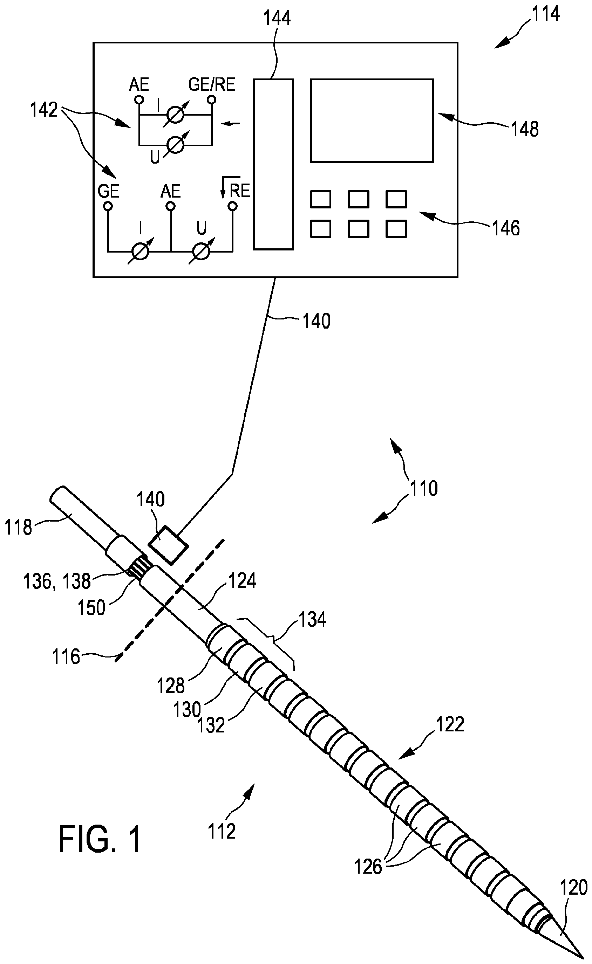

In Figur 1 ist in stark schematisierter Darstellung ein Ausführungsbeispiel eines Sensorsystems 110 zur Ermittlung einer Konzentration mindestens eines Analyten in einem Medium dargestellt. Das Sensorsystem 110 umfasst einen implantierbaren Sensor 112 und eine mit dem implantierbaren Sensor 112 verbundene Ansteuer- und Auswerteeinheit 114.

Im Folgenden sei, ohne den Schutzumfang der Erfindung einzuschränken, angenommen, dass es sich bei dem implantierbaren Sensor 112 um einen Glukosesensor handelt, welcher in ein Körpergewebe implantierbar ist. Dabei ist in Figur 1 symbolisch die Implantationstiefe durch die Hautlinie 116 dargestellt. Der implantierbare Sensor 112 ist von seiner Spitze bis zu dieser Hautlinie 116 in das Körpergewebe eingeführt. Die Implantationsnadel 118 wird nach dem Implantieren entfernt.

Der implantierbare Sensor 112 umfasst in diesem Beispiel als zentrales Element eine Implantationsnadel 118. Dabei ist diese Implantationsnadel 118 in diesem Ausführungsbeispiel als Akupunkturnadel ausgeführt und weist eine atraumatische Nadelspitze 120 auf, welche die Form einer Bleistiftspitze hat. Es ist zu erkennen, dass diese atraumatische Nadelspitze 120 keine zusätzlich scharfen Kanten aufweist, welche umliegendes Gewebe zerstören könnten.

Auf der Implantationsnadel 118 ist ein flexibles, schlauchförmiges Sensorelement 122 umfangsseitig aufgebracht, derart, dass die Implantationsnadel 118 mit ihrer atraumatischen Nadelspitze 120 am Implantationsende aus dem schlauchförmigen Sensorelement 122 herausragt. Die Implantationsnadel 118 ist dabei gleitend in dem schlauchförmigen Sensorelement 122 gelagert, so dass einerseits eine sichere Implantation durchgeführt werden kann, wobei andererseits nach der Implantation die Implantationsnadel 118 aus dem schlauchförmigen Sensorelement 122 herausgezogen werden kann. Dann verbleibt lediglich das schlauchförmige Sensorelement 122 innerhalb des Gewebes, d.h. rechts unterhalb der Hautlinie 116 in Figur 1. Alternativ kann die Implantationsnadel 118 auch lediglich teilweise aus dem schlauchförmigen Sensorelement 122 herausgezogen werden, um dann beispielsweise teilweise abgeknickt zu werden.

Das schlauchförmige Sensorelement 122 weist einen flexiblen, schlauchförmigen Körper 124 auf. Auf diesem schlauchförmigen Körper sind umfangsseitig Elektrodenringe 126 aufgebracht. Diese Elektrodenringe 126 sind hierbei entsprechend funktionalisiert, um jeweils eine Gegenelektrode 128, eine Referenzelektrode 130 und eine Arbeitselektrode 132 zu bilden. Somit bilden in diesem Ausführungsbeispiel jeweils drei Elektrodenringe 126 zusammen einen Einzelsensor 134, wobei die Einzelsensoren 134 des schlauchförmigen Sensorelements 122 prinzipiell voneinander unabhängig angesteuert und ausgewertet werden können. Die Gesamtzahl der Elektrodenringe 126 bildet also in diesem Ausführungsbeispiel gemäß Figur 1 ein ganzzahliges Vielfaches von Drei (in diesem Fall sind 15 Elektrodenringe vorgesehen).

Die Reihenfolge, Anzahl und Anordnung der Elektrodenringe 126 kann dabei von dem in Figur 1 dargestellten Ausführungsbeispiel abweichen. So können beispielsweise auch umgekehrte Reihenfolgen verwendet werden, wie beispielsweise Arbeitselektrode-Referenzelektrode-Gegenelektrode. Auch andere Anordnungen sind denkbar.

Alternativ zu den in Figur 1 dargestellten 3-Elektroden-Einzelsensoren 134 können auch Ausführungen verwendet werden, bei welchen lediglich zwei Elektrodenringe 126 einen Einzelsensor 134 bilden. In diesem Fall wäre vorzugsweise die Gesamtzahl der Elektrodenringe 126 ein Vielfaches von Zwei. Dieses Ausführungsbeispiel wäre besonders geeignet für eine Messmethode, welche Wechselströme einsetzt und welche lediglich jeweils eine Arbeitselektrode und eine Gegenelektrode oder eine kombinierte Gegenelektrode/Referenzelektrode benötigt.

Die Elektrodenringe 126 sind an einem der Spitze 120 abgewandten Ende des schlauchförmigen Sensorelements 122 über elektrische Kontakte 136, welche in diesem Ausführungsbeispiel auf einem Anschlusskontaktring 138 angeordnet sind, kontaktierbar und werden durch eine entsprechende elektrische Verbindung 140 kontaktiert und sind über die elektrische Verbindung 140 (beispielsweise ein Kabel oder auch eine drahtlose Verbindung) mit der Ansteuer- und Auswerteeinheit 114 verbunden.

Die Ansteuer- und Auswerteeinheit 114 ist dabei lediglich symbolisch dargestellt und umfasst eine Messvorrichtung 142 sowie eine Steuereinheit 144 (beispielsweise einen Mikrocomputer und/oder zusätzliche elektronische Komponenten), Bedienelemente 146 und eine oder mehrere Ausgabeeinheiten, beispielsweise ein Display 148. Alle Komponenten sind lediglich schematisch angedeutet und sind prinzipiell aus dem Stand der Technik bekannt. Die Messvorrichtung 142 ist mit verschiedenen Regel- und Messeinheiten ausgestattet, um beispielsweise Ströme I und Spannungen U zwischen den Elektroden (AE: Arbeitselektrode, GE: Gegenelektrode, RE: Referenzelektrode) zu messen und/oder zu regeln, so dass dem Fachmann bekannte elektrochemische Messungen zur Bestimmung der Glukosekonzentration durchführbar sind.

Die Ansteuer- und Auswerteeinheit 114 beziehungsweise die Messvorrichtung 142 und die Steuereinheit 144 können dabei auch weitere Funktionen umfassen, beispielsweise eine zumindest teilweise Auswertung und/oder Speicherung der gewonnenen Messdaten oder eine graphische Aufbereitung und/oder eine Datenbankfunktion. Auch kann eine "intelligente" Ansteuerung beziehungsweise Auswertung der Einzelsensoren 134 erfolgen, so dass diese beispielsweise nicht zeitlich parallel, sondern zum Beispiel nacheinander angesteuert und ausgewertet werden. Bei einem derartigen Multiplexing-Verfahren kann beispielsweise auch ein Einzelsensor 134 verwendet werden, während benachbarte Einzelsensoren 134 oder auch nur einzelne benachbarte Elektrodenringe 126 derart mit einem elektrischen Potential beaufschlagt werden, dass diese den gerade verwendeten Einzelsensor 134 abschirmen ("guard ring"-Funktion).

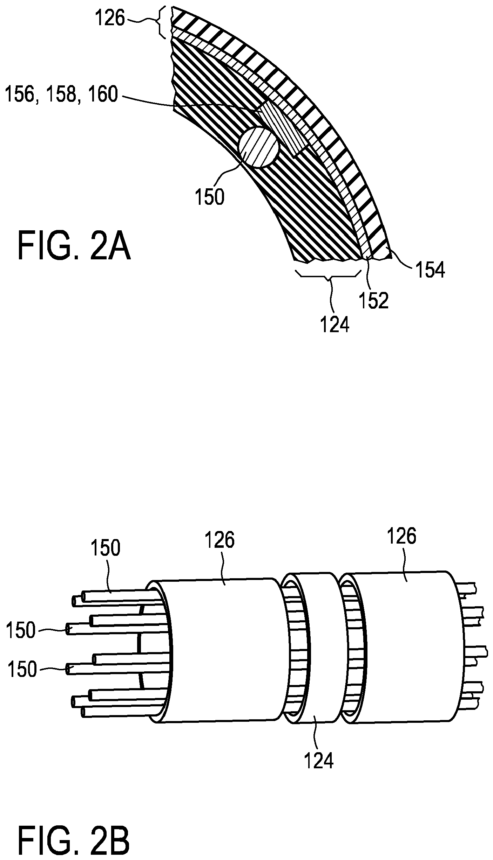

Der Aufbau und die Funktionsweise der Elektrodenringe 126 sind in den Figuren 2A und 2B schematisch dargestellt.

So ist aus der Teil-Querschnittsdarstellung in Figur 2A zu erkennen, dass in dem schlauchförmigen Körper 124 in axialer Richtung (d.h. parallel zur Implantationsnadel 118) Drähte 150 eingelassen sind. Diese Drähte 150 können, wie unten näher dargestellt, beispielsweise durch Koextrusion mit dem schlauchförmigen Körper 124 hergestellt werden, sind im schlauchförmigen Körper 124 näherungsweise gleichmäßig beabstandet angeordnet und sind gegeneinander elektrisch und galvanisch isoliert.

Auf den schlauchförmigen Körper 122 werden anschließend die Elektrodenringe 126 aufgebracht. In dem in Figur 2A und 2B dargestellten Ausführungsbeispiel weisen diese Elektrodenringe 126 jeweils zwei Schichten auf, nämlich einen elektrisch leitfähigen Kontaktring 152 und mindestens eine auf dem Kontaktring 152 aufgebrachte Funktionsschicht 154. Beispielsweise kann der leitfähige Kontaktring 152 aus einer leitfähigen Paste aufgebaut sein, welche ringförmig auf den schlauchförmigen Körper 124 aufgebracht wird. Die Funktionsschicht beziehungsweise Funktionsschichten 154 sind an die jeweilige Funktion des Elektrodenrings 126 angepasst und wurden oben bereits teilweise beschrieben. Zum Aufbau einer Arbeitselektrode können beispielsweise Braunstein, ein Puffer und ein Enzym in der Funktionsschicht 154 enthalten sein. Für den Aufbau einer Referenzelektrode können beispielsweise Silber und Silberchlorid-Partikel in der Funktionsschicht 154 enthalten sein. Eine Gegenelektrode kann beispielsweise keine Funktionsschicht 154 enthalten (in diesem Fall besteht der Elektrodenring 126 dann lediglich aus dem Kontaktring 152), oder es kann beispielsweise ein ähnlicher Aufbau wie für die Referenzelektrode verwendet werden.

Um den Kontaktring 152 mit einem darunter liegenden Draht 150 elektrisch zu verbinden, kann vorzugsweise vor dem Aufbringen des Kontaktrings 152 zunächst ein Durchkontaktierungsweg 156 in die oberste, den Draht 150 von der Oberfläche des schlauchförmigen Körpers 124 trennende Schicht gebohrt oder geschnitten werden. Beispielsweise können hierfür, wie oben beschrieben, mechanische Verfahren oder Laserablationsverfahren eingesetzt werden. Anschließend kann dieser Durchkontaktierungsweg 156 zumindest teilweise mit einem elektrisch leitfähigen Material 158 gefüllt werden. Auf dieses elektrisch leitfähige Material 158 wird dann der Kontaktring 152 aufgebracht. Dabei kann das Einbringen des elektrisch leitfähigen Materials 158 und die Herstellung des Kontaktrings 152 auch in ein und demselben Arbeitsschritt erfolgen, da in beiden Schritten beispielsweise eine leitfähige Paste verwendet wird.

Anschließend kann beispielsweise der Kontaktring 152 und/oder das elektrisch leitfähige Material 158 ausgehärtet werden, um dann in einem ähnlichen Verfahren die Funktionsschicht 152 aufzubringen. Es sei darauf hingewiesen, dass anstelle des vereinfacht dargestellten Aufbaus auch ein komplexerer, mehrschichtiger Aufbau möglich ist.

In der perspektivischen Darstellung in Figur 2B ist dargestellt, wie die Elektrodenringe 126 das Gerüst der in diesem Fall gleichmäßig verteilten Drähte 150 umfangsseitig umgeben. Der schlauchförmige Körper 124 ist dabei lediglich ansatzweise dargestellt, um zu zeigen, dass die einzelnen Elektrodenringe 126 elektrisch voneinander isoliert sind. Alternativ oder zusätzlich können entlang der Längsachse des implantierbaren Sensors 112 auch zwischen den einzelnen Elektrodenringen 126 zusätzliche Isolierungen vorgesehen sein, beispielsweise in Form zusätzlicher isolierender Ringe zwischen diesen Elektrodenringen 126, die beispielsweise auch das Aufbringen der einzelnen Elektrodenringe und deren Isolierung voneinander erleichtern.

Auf diese Weise kann beispielsweise jedem der Elektrodenringe 126 ein bestimmter Draht 150 zugeordnet werden, indem Elektrodenring 126 und Draht 150 durch die Durchkontaktierung 160 miteinander verbunden sind. An dem elektrischen Kontakten 136 an der Spitze 120 abgewandten Ende des schlauchförmigen Sensorelements 122 (siehe Figur 1) können beispielsweise wiederum Anschlusskontaktringe erzeugt werden, welche mit beispielsweise jeweils einem der Drähte 150 verbunden sind.

Alternativ kann der Anschlusskontaktring 138 auch einfach dadurch erzeugt werden, dass in diesem Bereich die Drähte 150 freigelegt werden, indem wiederum die Materialschicht des schlauchförmigen Körpers 124 zwischen den Drähten 150 und der Oberfläche des schlauchförmigen Körpers 124 entfernt wird. Beispielsweise können für diese Entfernung wiederum mechanische Abtragverfahren verwendet werden, oder beispielsweise auch ein Laserablationsverfahren.

Zur Kontaktierung der einzelnen Drähte 150 können dann beispielsweise in diesem Bereich Verfahren angewandt werden, welche beispielsweise aus der Display-Technologie bekannt sind, wie beispielsweise um den schlauchförmigen Körper 124 herumgewickelte Flexverbinder, Leitgummis oder Zebra-Verbinder. Beispielsweise kann zu diesem Zweck auch die elektrische Verbindung 140 als ringförmiges Element ausgestaltet sein, welche vom der Spitze 120 abgewandten Ende des schlauchförmigen Sensorelements 122 auf den Anschlusskontaktring 138 aufgeschoben wird. Zur Zuordnung der einzelnen Elektrodentypen beziehungsweise Drähte können beispielsweise die Drähte 150 in einer definierten Reihenfolge mit den Elektrodenringen 126 verbunden sein. Auch eine Zuordnung wäre denkbar, bei welcher in der elektrischen Verbindung 140 oder an einem entfernten Ort ein Revolver-artiger Drehmechanismus integriert ist, um erst am vereinzelten implantierbaren Sensor 112 eine Zuordnung vorzunehmen. Auch eine elektronische Zuordnung ist denkbar.

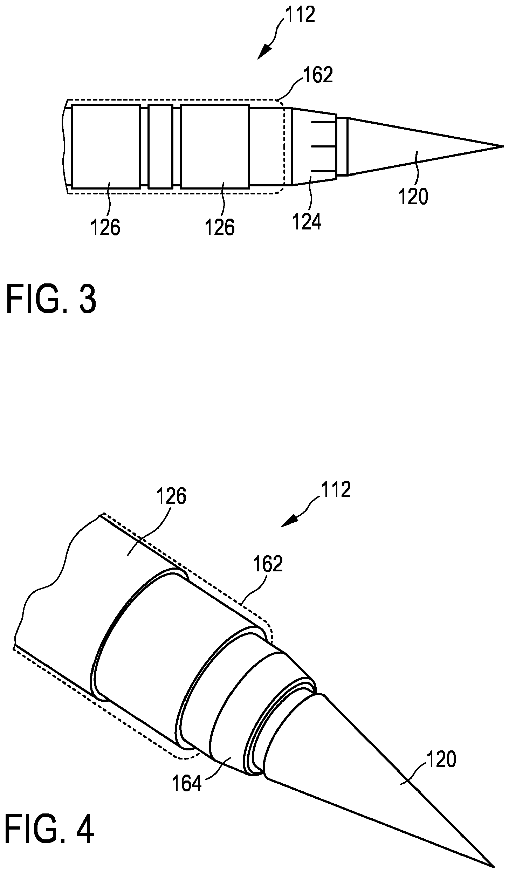

In den Figuren 3 und 4 sind zwei verschiedene Ausführungsformen der Spitzen des implantierbaren Sensors 112 dargestellt. Dabei ist erkennbar, dass vorzugsweise (was in Figur 1 zur Vereinfachung nicht dargestellt ist) der Bereich des schlauchförmigen Sensorelements 122, in welchem die Elektrodenringe 126 aufgebracht sind, zusätzlich von einer biokompatiblen, für den Analyten (beispielsweise Glukose) durchlässigen Membran 162 umgeben ist. Diese Membran kann beispielsweise eine Polymermembran sein und kann einerseits dem Schutz des umgebenden Gewebes vor toxischen Bestandteilen der Elektrodenringe 126 dienen und kann beispielsweise auch einen Stromfluss beim elektrochemischen Messverfahren gezielt begrenzen. Die Membranschicht 162 kann beispielsweise in einem Tauch- oder Sprühverfahren aufgebracht werden.

Die beiden Ausführungsbeispiele der implantierbaren Sensoren 112 gemäß den Figuren 3 und 4 unterscheiden sich im Wesentlichen dadurch, dass in Figur 3 das der Nadelspitze 120 zuweisende Ende des schlauchförmigen Körpers 124 frei liegt. Diese Ausführungsform hat unter Umständen den Nachteil, dass Drähte 150 in diesem Bereich entweder mit der Implantationsnadel 118 in Kontakt kommen oder ungeschützt mit dem umgebenden Körpergewebe, wobei beispielsweise elektrochemische Reaktionen ausgelöst werden könnten und wodurch die Messergebnisse der elektrochemischen Analyt-Konzentrationsbestimmung verfälscht werden könnten. Im Gegensatz hierzu ist in der Ausführungsform gemäß Figur 4 das der Spitze 120 der Implantationsnadel 118 zuweisende Ende des schlauchförmigen Sensorelements 122 mit einem isolierenden Lack 164 überzogen. Beispielsweise kann dies ebenfalls wieder leicht großtechnisch durch ein Tauch- oder Sprühverfahren erfolgen. Auf diese Weise sind auch die Enden der Drähte 150 sicher gegeneinander, gegen die Implantationsnadel 118 und gegen das umgebende Körpergewebe isoliert.

Der implantierbare Sensor 112 gemäß den vorhergehenden beschriebenen Ausführungsbeispielen lässt sich auf einfache und kostengünstige Weise großtechnisch und mit hohem Durchsatz herstellen. Diese einfache Herstellung ist eine wesentliche Voraussetzung für die Verwendung als Einweg-Sensor, welcher nach einer Benutzungsdauer von wenigen Stunden oder Tagen entsorgt werden kann.

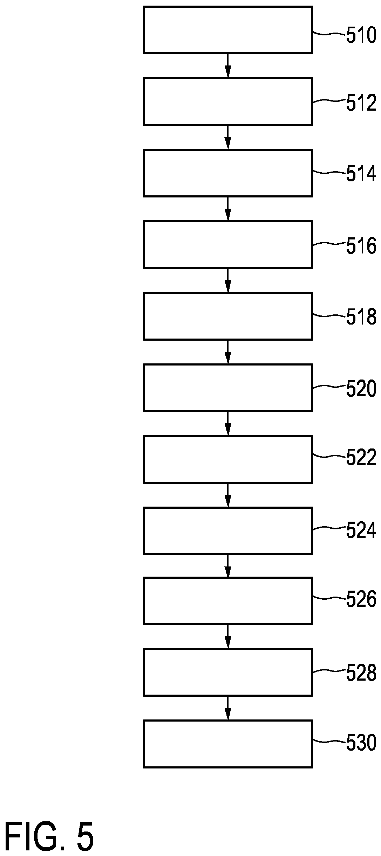

In Figur 5 ist ein mögliches Ausführungsbeispiel eines Herstellungsverfahrens zur Herstellung des implantierbaren Sensors 112 schematisch dargestellt. Es sei darauf hingewiesen, dass die dargestellten Verfahrensschritte vorzugsweise, jedoch nicht notwendigerweise in der dargestellten Reihenfolge durchgeführt werden sollten, dass einzelne oder mehrere Verfahrensschritte auch unabhängig voneinander, d.h. zeitgleich oder zeitlich überlappend durchgeführt werden können, und dass einzelne Verfahrensschritte auch wiederholt durchgeführt werden können.

In einem ersten Verfahrensschritt (510) wird der schlauchförmige Körper 124 hergestellt. Dieser Schritt kann, wie oben dargestellt, beispielsweise durch Koextrusion des Schlauchmaterials mit den Drähten 150 erfolgen. Beispielsweise kann der Schlauch selbst einen äußeren Durchmesser von 0,8 mm und eine Wandstärke von 0,2 mm aufweisen. Beispielsweise können in dem Schlauch 9 bis 15 Drähte eingebettet sein.

Anschließend werden in Verfahrensschritt 512 die Durchkontaktierungswege 156 hergestellt. Diese werden dann in Verfahrensschritt 514 mit dem elektrisch leitfähigen Material 158 gefüllt.

In Verfahrensschritt 516 werden dann die Kontaktringe 152 aufgebracht. Dies kann, wie oben beschrieben, beispielsweise durch ein Tampon-Transferverfahren (mit einer großen Rolle auf einem kleinen Zylinder) durch ein Offset-Druckverfahren, durch ein Jet-Verfahren (zum Beispiel ein Piezo-Verfahren oder andere Jet-Verfahren, wie beispielsweise ein Verfahren, bei welchem ein Jet durch einen mechanischen Impuls auf eine Kapillare erzeugt wird), durch ein elektrostatisches Verfahren, durch ein Elektrospray-Verfahren (wie es beispielsweise aus der Aluminium-Lackindustrie bekannt ist) oder durch ein Xerox-Verfahren erfolgen.

Eine alternative Methode zur Herstellung der Kontaktringe 152 beziehungsweise der gesamten Elektrodenringe 126 besteht darin, dass diese auf einem flachen, flexiblen Träger präpariert werden. Dieser flache Träger erlaubt die Verwendung einfacher, großflächiger Herstellungsmethoden, welche üblicherweise auf ebenen Substraten durchgeführt werden. Anschließend können diese flachen Träger um den schlauchförmigen Körper 124 herum montiert werden. Beispielsweise kann der flexible, flache Träger ein flaches Trägerband umfassen, auf welchem die benötigte Anzahl an Elektrodenstreifen aufgebracht wird. Dieses flache Trägerband kann dann beispielsweise zurechtgeschnitten werden und als langes "Etikett" auf den Hohlzylinder des schlauchförmigen Körpers 124 aufgeklebt werden. Die Kontaktierung der auf diese Weise erzeugten Kontaktringe 152 beziehungsweise Elektrodenringe 126 kann wiederum auf die oben beschriebene Weise erfolgen, d.h. beispielsweise durch anschließendes Erzeugen eines Durchkontaktierungsweges 156, eines Füllens mit elektrisch leitfähigem Material 158 und anschließenden Aushärten.

Die Herstellungsmethoden können auch kombiniert werden, indem zum Beispiel die leitfähigen Schichten auf einem flachen, separaten Trägermaterial erzeugt werden, dann auf den schlauchförmigen Körper 124 aufgebracht werden, um dann anschließend auf den Kontaktringen 152 die Funktionsschichten 154 aufzubringen.

Das Aufbringen der Funktionsschichten 154 ist in Figur 5 symbolisch mit der Bezugsziffer 518 dargestellt.

Anschließend erfolgt in Verfahrensschritt 520 ein Schneiden des derart erzeugten schlauchförmigen Elements auf die für das schlauchförmige Sensorelement 122 benötigte Länge. Beispielsweise können Abschnitte von 18 mm Länge zurechtgeschnitten werden.

Anschließend erfolgt in Verfahrensschritt 522 ein Isolieren des Sensorelements, was - wie oben beschrieben - beispielsweise durch Eintauchen in einen isolierenden Lack 164 erfolgen kann.

In Verfahrensschritt 524 wird der Anschlusskontaktring 138 erzeugt. Dies kann, wie oben beschrieben, beispielsweise durch zumindest teilweises Freilegen der Drähte 150 erfolgen. Auch andere Methoden wurden oben beschrieben.

In Schritt 526 wird zumindest ein Teil des schlauchförmigen Sensorelements 122 (insbesondere vorzugsweise der Teil, auf welchem sich Elektrodenringe 126 befinden) mit der Analyt-permeablen und biokompatiblen Membranschicht 162 beschichtet.

Anschließend kann in Verfahrensschritt 528 ein Kontrollschritt erfolgen, bei welchem beispielsweise eine Grobkontrolle der Funktion des schlauchförmigen Sensorelements 122 erfolgt.

Anschließend kann in Verfahrensschritt 530 die Implantationsnadel 118 in den schlauchförmigen Körper 124 eingeschoben werden. Um eine Kontamination dieser Nadel während der Herstellung zu vermeiden, erfolgt dieses Einführen vorzugsweise erst nach Herstellung des schlauchförmigen Sensorelements 122. Während der Herstellung des schlauchförmigen Sensorelements kann statt der Nadel auch ein anderes stabförmiges Trägerelement in das Innere des schlauchförmigen Körpers 124 eingeschoben werden, welches anschließend durch die Implantationsnadel 118 ersetzt wird.

**Bezugszeichenliste**

| | | | |
|---|---|---|---|
| 110 | Sensorsystem | 520 | Schneiden |
| 112 | Implantierbarer Sensor | 522 | Isolierung des Endes des Sensorelements |
| 114 | Ansteuer- und Auswerteeinheit | | |
| 116 | Hautlinie | 524 | Erzeugen des Anschlusskontaktrings |
| 118 | Implantationsnadel | 526 | Beschichten mit Membranschicht |
| 120 | Nadelspitze | 528 | Funktionskontrolle |
| 122 | schlauchförmiges Sensorelement | 530 | Einfügen der Implantationsnadel |
| 124 | schlauchförmiger Körper | | |
| 126 | Elektrodenringe | | |
| 128 | Gegenelektrode | | |
| 130 | Referenzelektrode | | |
| 132 | Arbeitselektrode | | |
| 134 | Einzelsensor | | |
| 136 | elektrische Kontakte | | |
| 138 | Anschlusskontaktring | | |
| 140 | elektrische Verbindung | | |
| 142 | Messvorrichtung | | |
| 144 | Steuereinheit | | |
| 146 | Bedienelemente | | |
| 148 | Display | | |
| 150 | Drähte | | |
| 152 | Kontaktring | | |
| 154 | Funktionsschicht | | |
| 156 | Durchkontaktierungsweg | | |
| 158 | elektrisch leitfähiges Material | | |
| 160 | Durchkontaktierung | | |
| 162 | Membranschicht | | |
| 164 | iso lierender Lack | | |
| | | | |
| 510 | Herstellen des schlauchförmigen Körpers | | |
| 512 | Herstellen der Durchkontaktierungswege | | |
| 514 | Füllen der Durchkontaktierungswege | | |
| 516 | Aufbringen der Kontaktringe | | |
| 518 | Aufbringen der Funktionsschichten | | |

## Patentansprüche

1. Implantierbarer Sensor (112) zur Ermittlung einer Konzentration mindestens eines Analyten in einem Medium, insbesondere einem Körpergewebe und/oder einer Körperflüssigkeit, wobei der implantierbare Sensor (112) ein flexibles, schlauchförmiges Sensorelement (122) aufweist, wobei das schlauchförmige Sensorelement (122) einen flexiblen, schlauchförmigen Körper (124) aufweist, wobei mindestens zwei Elektrodenringe (126) zur elektrochemischen Bestimmung der Konzentration des Analyten auf den schlauchförmigen Körper (124) aufgebracht sind, **dadurch gekennzeichnet dass** die mindestens zwei Elektrodenringe (126) jeweils alternierend beschaltet sind.

2. Implantierbarer Sensor (112) nach dem vorhergehenden Anspruch, wobei in dem schlauchförmigen Körper (124) elektrisch und/oder galvanisch voneinander isolierte Zuleitungen (150) für die mindestens zwei Elektrodenringe (126) eingebettet sind.

3. Implantierbarer Sensor (112) nach dem vorhergehenden Anspruch, wobei die Elektrodenringe (126) über den Körper (124) zumindest teilweise durchdringende elektrische Durchkontaktierungen (160) elektrisch mit den Zuleitungen (150) verbunden sind.

4. Implantierbarer Sensor (112) nach einem der vorhergehenden Ansprüche, wobei an einem dem Implantationsende abgewandten Ende des Sensorelements (122) elektrische Kontakte (136) zur Kontaktierung der Elektrodenringe (126) vorgesehen sind.

5. Implantierbarer Sensor (112) nach einem der vorhergehenden Ansprüche, wobei die Elektrodenringe (126) jeweils mindestens einen dem schlauchförmigen Körper (124) zugewandten elektrisch leitfähigen Kontaktring (152) und jeweils mindestens eine auf dem Kontaktring (152) aufgebrachte Funktionsschicht (154) umfassen.

6. Implantierbarer Sensor (112) nach einem der vorhergehenden Ansprüche, wobei die Elektrodenringe (126) auf der dem schlauchförmigen Körper (124) abgewandten Seite zumindest teilweise mit mindestens einer Membranschicht (162) umgeben sind, wobei die mindestens eine Membranschicht (162) zumindest teildurchlässig ist für den mindestens einen Analyten.

7. Implantierbarer Sensor (112) nach einem der vorhergehenden Ansprüche, wobei ein dem Implantationsende zugewandtes Ende des schlauchförmigen Sensorelements (122) mit einer elektrischen Isolierung abgeschlossen ist, insbesondere mit einem isolierenden Lack (164).

8. Implantierbarer Sensor (112) nach einem der vorhergehenden Ansprüche, weiterhin umfassend eine Implantationsnadel (118), welche in dem schlauchförmigen Körper (124) gleitbar gelagert ist, derart, dass die Implantationsnadel (118) nach Implantation des Sensors (112) in ein Körpergewebe zumindest teilweise aus dem schlauchförmigen Körper (124) herausziehbar ist.

9. Implantierbarer Sensor (112) nach dem vorhergehenden Anspruch, wobei die Implantationsnadel (118) eine Akupunkturnadel ist, insbesondere eine Akupunkturnadel mit einem Durchmesser zwischen 0,40 mm und 0,7 mm.

10. Implantierbarer Sensor (112) nach einem der beiden vorhergehenden Ansprüche, wobei die Implantationsnadel (118) eine Nadel mit einer Spitze (120) ist, welche zumindest teilweise aus einem biokompatiblen, enzymatisch abbaubarem Material besteht, derart, dass die Spitze (120) sich nach Implantation in ein Körpergewebe zumindest teilweise verrundet.

11. Sensorsystem zur Ermittlung einer Konzentration mindestens eines Analyten in einem Medium, insbesondere einem Körpergewebe und/oder einer Körperflüssigkeit, umfassend mindestens einen implantierbaren Sensor (112) nach einem der vorhergehenden Ansprüche und weiterhin umfassend eine Ansteuer- und Auswerteeinheit (114), welche mit den Elektrodenringen (126) verbunden ist und welche eingerichtet ist, um eine elektrochemische Messung zur Bestimmung der Konzentration des Analyten durchzuführen.

12. Verfahren zur Herstellung eines implantierbaren Sensors (112) zur Ermittlung einer Konzentration mindestens eines Analyten in einem Medium, insbesondere einem Körpergewebe und/oder einer Körperflüssigkeit, wobei das Verfahren die folgenden Schritte umfasst:
- ein schlauchförmiger Körper (124) für ein schlauchförmiges Sensorelement (122) wird hergestellt; und
- mindestens zwei Elektrodenringe (126) zur elektrochemischen Bestimmung der Konzentration des Analyten werden auf den Körper (124) aufgebracht,
**dadurch gekennzeichnet dass** die mindestens zwei Elektrodenringe (126) jeweils alternierend beschaltet sind.

13. Verfahren nach dem vorhergehenden Anspruch, mit zusätzlich folgendem Schritt:
- in den schlauchförmigen Körper (124) werden elektrisch und/oder galvanisch voneinander isolierte Zuleitungen (150) für die mindestens zwei Elektrodenringe (126) eingebettet.

14. Verfahren nach dem vorhergehenden Anspruch, wobei die Einbettung in einem Verfahrensschritt mit der Herstellung des schlauchförmigen Körpers (124) erfolgt, insbesondere durch Koextrusion.

15. Verfahren nach einem der zwei vorhergehenden Ansprüche, wobei eine Durchkontaktierung hergestellt wird, um die Elektrodenringe (126) elektrisch mit den Zuleitungen (150) zu verbinden.

16. Verfahren nach dem vorhergehenden Anspruch, wobei zur Durchkontaktierung ein Durchkontaktierungsweg (156) durch mindestens eine Materialschicht zwischen den Elektrodenringen (126) und den Zuleitungen (150) erzeugt wird und wobei anschließend der Durchkontaktierungsweg (156) zumindest teilweise mit mindestens einem elektrisch leitfähigen Material (158) gefüllt wird.

17. Verfahren nach einem der vorhergehenden Verfahrensansprüche, wobei beim Herstellen der Elektrodenringe (126) jeweils zunächst mindestens ein dem schlauchförmigen Körper (124) zugewandter elektrisch leitfähiger Kontaktring (152) erzeugt wird und anschließend jeweils mindestens eine auf dem Kontaktring (152) aufgebrachte Funktionsschicht (154).

18. Verfahren nach dem vorhergehenden Anspruch, wobei beim Aufbringen der Kontaktringe (152) und/oder der Funktionsschichten (154) jeweils mindestens eines der folgenden Verfahren zum strukturierten Aufbringen einer Schicht verwendet wird: ein Laminierverfahren; ein Sprühverfahren; ein Inkjet-Verfahren, insbesondere ein Piezo- oder Thermojet-Verfahren; ein Kontaktdruckverfahren, insbesondere ein Tampondruckverfahren, ein Offset-Verfahren; ein elektrostatisches Druckverfahren.

19. Verfahren nach einem der beiden vorhergehenden Ansprüche, wobei beim Aufbringen der Kontaktringe (152) und/oder der Funktionsschichten (154) zumindest ein Kontaktring (152) und/oder zumindest eine Funktionsschicht (154) auf ein flexibles Trägersubstrat aufgebracht werden, wobei anschließend das flexible Trägersubstrat auf den schlauchförmigen Körper (124) aufgebracht wird.

20. Verfahren nach einem der vorhergehenden Verfahrensansprüche, mit zusätzlich folgendem Schritt:
- die Elektrodenringe (126) werden auf der dem schlauchförmigen Körper (124) abgewandten Seite zumindest teilweise mit mindestens einer Membranschicht (162) umgeben, wobei die mindestens eine Membranschicht (162) zumindest teildurchlässig ist für den mindestens einen Analyten.

21. Verfahren nach einem der vorhergehenden Verfahrensansprüche, mit zusätzlich folgendem Schritt:
- an einem dem Implantationsende abgewandten Ende des Sensorelements (122) werden elektrische Kontakte (136) zur Kontaktierung der Elektrodenringe (126) erzeugt.

22. Verfahren nach einem der vorhergehenden Verfahrensansprüche, mit zusätzlich folgendem Verfahrensschritt:
- ein dem Implantationsende zugewandtes Ende des schlauchförmigen Sensorelements (122) wird mit einer elektrischen Isolierung abgeschlossen, insbesondere mit einem isolierenden Lack (164).

23. Verfahren nach einem der vorhergehenden Verfahrensansprüche, mit zusätzlich folgendem Verfahrensschritt:
- in den schlauchförmigen Körper (124) wird eine Implantationsnadel (118) eingebracht.

## Claims

1. Implantable sensor (112) for determining a concentration of at least one analyte in a medium, particularly in a body tissue and/or a body fluid, wherein the implantable sensor (112) has a flexible, tubular sensor element (122), wherein the tubular sensor element (122) has a flexible, tubular body (124), wherein at least two electrode rings (126) for the electrochemical determination of the concentration of the analyte are mounted on the tubular body (124), **characterized in that** the at least two electrode rings (126) are in each case wired in an alternating manner.

2. Implantable sensor (112) according to the preceding claim, wherein electrically and/or galvanically insulated supply lines (150) for the at least two electrode rings (126) are embedded in the tubular body (124).

3. Implantable sensor (112) according to the preceding claim, wherein the electrode rings (126) are electrically connected to the supply lines (150) by electrical plated through-holes (160) which extend at least partially through the body (124).

4. Implantable sensor (112) according to one of the preceding claims, wherein electrical contacts (136) for the contacting of the electrode rings (126) are provided at an end of the sensor element (122) directed away from the implantation end.

5. Implantable sensor (112) according to one of the preceding claims, wherein the electrode rings (126) comprise in each case at least one electrically conductive contact ring (152), directed towards the tubular body (124), and in each case at least one function layer (154) applied to the contact ring (152) .

6. Implantable sensor (112) according to one of the preceding claims, wherein the electrode rings (126), on the side directed away from the tubular body (124), are at least partially surrounded by at least one membrane layer (162), and wherein the at least one membrane layer (162) is at least partially permeable to the at least one analyte.

7. Implantable sensor (112) according to one of the preceding claims, wherein an end of the tubular sensor element (122), directed towards the implantation end, is closed off with an electrical insulation, in particular with an insulating coating (164) .

8. Implantable sensor (112) according to one of the preceding claims, further comprising an implantation needle (118), which is mounted slidably in the tubular body (124) in such a manner that the implantation needle (118), after implantation of the sensor (112) in a body tissue, can be at least partially withdrawn from the tubular body (124).

9. Implantable sensor (112) according to the preceding claim, wherein the implantation needle (118) is an acupuncture needle, in particular an acupuncture needle with a diameter of between 0.40 mm and 0.7 mm.

10. Implantable sensor (112) according to one of the two preceding claims, wherein the implantation needle (118) is a needle with a tip (120) which at least partially consists of a biocompatible, enzymatically degradable material, such that the tip (120) becomes at least partially rounded after implantation in a body tissue.

11. Sensor system for determining a concentration of at least one analyte in a medium, particularly in a body tissue and/or a body fluid, comprising at least one implantable sensor (112) according to one of the preceding claims, and further comprising a control and evaluation unit (114) which is connected to the electrode rings (126) and which is configured to carry out an electrochemical measurement in order to determine the concentration of the analyte.

12. Method for producing an implantable sensor (112) for determining a concentration of at least one analyte in a medium, particularly in a body tissue and/or a body fluid, wherein the method comprises the following steps:
- a tubular body (124) for a tubular sensor element (122) is produced; and
- at least two electrode rings (126) for electrochemical determination of the concentration of the analyte are applied to the body (124),
**characterized in that** the at least two electrode rings (126) are in each case wired in an alternating manner.

13. Method according to the preceding claim, additionally with the following step:
- electrically and/or galvanically insulated supply lines (150) for the at least two electrode rings (126) are embedded in the tubular body (124) .

14. Method according to the preceding claim, wherein the embedding takes place in a method step comprising the production of the tubular body (124), particularly by co-extrusion.

15. Method according to one of the two preceding claims, wherein a plated through-hole is produced for connecting the electrode rings (126) electrically to the supply lines (150).

16. Method according to the preceding claim, wherein the plated through-hole is produced by generating a via (156) through at least one material layer between the electrode rings (126) and the supply lines (150), and wherein the via (156) is then at least partially filled with at least one electrically conductive material (158).

17. Method according to one of the preceding method claims, wherein, for producing the electrode rings (126), in each case at least one electrically conductive contact ring (152) directed towards the tubular body (124) is, firstly, generated, and in each case at least one function layer (154) is, subsequently, applied to the contact ring (152).

18. Method according to the preceding claim, wherein the contact rings (152) and/or the function layers (154) are applied using in each case at least one of the following methods for structured application of a layer: a laminating method; a spraying method; an inkjet method, in particular a piezo or thermo jet method; a contact printing method, in particular a pad printing method, an offset method; an electrostatic printing method.

19. Method according to one of the two preceding claims, wherein the application of the contact rings (152) and/or of the function layers (154) involves at least one contact ring (152) and/or at least one function layer (154) being applied to a flexible carrier substrate, wherein the flexible carrier substrate is, subsequently, applied to the tubular body (124).

20. Method according to one of the preceding method claims, additionally with the following step:
- the electrode rings (126), on the side directed away from the tubular body (124), are at least partially surrounded by at least one membrane layer (162), wherein the at least one membrane layer (162) is at least partially permeable to the at least one analyte.

21. Method according to one of the preceding method claims, additionally with the following step:
- electrical contacts (136) for the contacting of the electrode rings (126) are generated at an end of the sensor element (122) directed away from the implantation end.

22. Method according to one of the preceding method claims, additionally with the following method step:
- an end of the tubular sensor element (122) directed towards the implantation end is closed off with an electrical insulation, in particular with an insulating coating (164).

23. Method according to one of the preceding method claims, additionally with the following method step:
- an implantation needle (118) is introduced into the tubular body (124).

## Revendications

1. Capteur implantable (112) destiné à la détermination d'une concentration d'au moins un analyte dans un milieu, en particulier un tissu corporel et/ou un liquide corporel, dans lequel le capteur implantable (112) présente un élément de capteur flexible tubulaire (122), dans lequel l'élément de capteur tubulaire (122) présente un corps tubulaire flexible (124), dans lequel au moins deux anneaux d'électrode (126) sont montés sur le corps tubulaire (124) pour la détermination de la concentration de l'analyte,
**caractérisé en ce que** lesdits au moins deux anneaux d'électrode (126) sont respectivement connectés en alternance.

2. Capteur implantable (112) selon la revendication précédente, dans lequel des conducteurs d'alimentation (150) isolés électriquement et/ou galvaniquement les uns des autres pour lesdits au moins deux anneaux d'électrode (126) sont incorporés dans le corps tubulaire (124).

3. Capteur implantable (112) selon la revendication précédente, dans lequel les anneaux d'électrode (126) sont raccordés électriquement aux conducteurs d'alimentation (150) par des contacts électriques pénétrants (160) qui traversent au moins en partie le corps (124) .

4. Capteur implantable (112) selon l'une quelconque des revendications précédentes, dans lequel il est prévu à une extrémité de l'élément de capteur (122) opposée à l'extrémité d'implantation des contacts électriques (136) pour la mise en contact des anneaux d'électrode (126) .

5. Capteur implantable (112) selon l'une quelconque des revendications précédentes, dans lequel les anneaux d'électrode (126) comprennent respectivement au moins un anneau de contact électriquement conducteur (152) tourné vers le corps tubulaire (124) et respectivement au moins une couche fonctionnelle déposée sur l'anneau de contact (152) .

6. Capteur implantable (112) selon l'une quelconque des revendications précédentes, dans lequel les anneaux d'électrode (126) sont entourés sur le côté opposé au corps tubulaire (124) au moins partiellement avec au moins une couche de membrane (162), dans lequel ladite au moins une couche de membrane (162) est au moins partiellement perméable audit au moins un analyte.

7. Capteur implantable (112) selon l'une quelconque des revendications précédentes, dans lequel une extrémité de l'élément de capteur tubulaire (122) tournée vers l'extrémité d'implantation est fermée avec une isolation électrique, en particulier avec une laque isolante (164).

8. Capteur implantable (112) selon l'une quelconque des revendications précédentes, comprenant en outre une aiguille d'implantation (118), qui est placée de façon glissante dans le corps tubulaire (124), de telle manière que l'aiguille d'implantation (118) puisse être retirée au moins partiellement hors du corps tubulaire (124) après l'implantation du capteur (112) dans un tissu corporel.

9. Capteur implantable (112) selon l'une quelconque des revendications précédentes, dans lequel l'aiguille d'implantation (118) est une aiguille d'acupuncture, en particulier une aiguille d'acupuncture d'un diamètre compris entre 0,40 mm et 0,7 mm.

10. Capteur implantable (112) selon l'une quelconque des deux revendications précédentes, dans lequel l'aiguille d'implantation (118) est une aiguille avec une pointe (120), qui est constituée au moins en partie d'un matériau biocompatible, dégradable par voie enzymatique, de telle manière que la pointe (120) s'arrondisse au moins partiellement après implantation dans un tissu corporel.

11. Système de capteur destiné à la détermination d'une concentration d'au moins un analyte dans un milieu, en particulier un tissu corporel et/ou un liquide corporel, comprenant au moins un capteur implantable (112) selon l'une quelconque des revendications précédentes et comprenant en outre une unité de commande et d'évaluation (114), qui est reliée aux anneaux d'électrode (126) et qui est conçue pour effectuer une mesure électrochimique pour la détermination de la concentration de l'analyte.

12. Procédé de fabrication d'un capteur implantable (112) destiné à la détermination d'une concentration d'au moins un analyte dans un milieu, en particulier un tissu corporel et/ou un liquide corporel, dans lequel le procédé comprend les étapes suivantes:
- on fabrique un corps tubulaire (124) pour un élément de capteur tubulaire (122); et
- on monte au moins deux anneaux d'électrode (126) destinés à la détermination électrochimique de la concentration de l'analyte sur le corps (124),
**caractérisé en ce que** lesdits au moins deux anneaux d'électrode (126) sont respectivement connectés en alternance.

13. Procédé selon la revendication précédente, comportant en outre l'étape suivante:
- on incorpore dans le corps tubulaire (124) des conducteurs d'alimentation (150) électriquement et/ou galvaniquement isolés les uns des autres pour lesdits au moins deux anneaux d'électrode (126).

14. Procédé selon la revendication précédente, dans lequel on effectue l'incorporation dans une étape de procédé avec la fabrication du corps tubulaire (124), en particulier par co-extrusion.

15. Procédé selon l'une quelconque des deux revendications précédentes, dans lequel on fabrique un contact pénétrant, pour relier électriquement les anneaux d'électrode (126) aux conducteurs d'alimentation (150).

16. Procédé selon la revendication précédente, dans lequel pour le contact pénétrant on produit un chemin de contact pénétrant (156) à travers au moins une couche de matière entre les anneaux d'électrode (126) et les conducteurs d'alimentation (150) et dans lequel on remplit ensuite le chemin de contact pénétrant (156) au moins en partie avec au moins un matériau électriquement conducteur (158).

17. Procédé selon l'une quelconque des revendications de procédé précédentes, dans lequel lors de la fabrication des anneaux d'électrode (126) on produit chaque fois d'abord au moins un anneau de contact électriquement conducteur (152) tourné vers le corps tubulaire (124) et ensuite chaque fois au moins une couche fonctionnelle (154) déposée sur l'anneau de contact (152).

18. Procédé selon la revendication précédente, dans lequel lors du dépôt des anneaux de contact (152) et/ou des couches fonctionnelles (154), on utilise chaque fois un des procédés suivants pour le dépôt structuré d'une couche: un procédé de stratification; un procédé de projection; un procédé à jet d'encre, en particulier un procédé à piézojet ou à thermojet; un procédé d'impression par contact, en particulier un procédé d'impression au tampon, un procédé offset; un procédé d'impression électrostatique.

19. Procédé selon l'une quelconque des deux revendications précédentes, dans lequel lors du dépôt des anneaux de contact (152) et/ou des couches fonctionnelles (154) on dépose au moins un anneau de contact (152) et/ou au moins une couche fonctionnelle (154) sur un substrat de support flexible, dans lequel on dépose ensuite le substrat de support flexible sur le corps tubulaire (124) .

20. Procédé selon l'une quelconque des revendications de procédé précédentes, comportant en outre l'étape suivante:
- on entoure les anneaux d'électrode (126), sur le côté opposé au corps tubulaire (124), au moins en partie avec au moins une couche de membrane (162), dans lequel ladite au moins une couche de membrane (162) est au moins partiellement perméable audit au moins un analyte.

21. Procédé selon l'une quelconque des revendications de procédé précédentes, comportant en outre l'étape suivante:
- on produit à une extrémité de l'élément de capteur (122) opposée à l'extrémité d'implantation des contacts électriques (136) destinés à la mise en contact des anneaux d'électrode (126).

22. Procédé selon l'une quelconque des revendications de procédé précédentes, comportant en outre l'étape suivante:
- on ferme une extrémité de l'élément de capteur tubulaire (122) tournée vers l'extrémité d'implantation avec une isolation électrique, en particulier avec une laque isolante (164).

23. Procédé selon l'une quelconque des revendications de procédé précédentes, comportant en outre l'étape suivante:
- on introduit une aiguille d'implantation (118) dans le corps tubulaire (124).
